Europäisches Patentamt

European Patent Office

Office européen des brevets

(11) Publication number: **0 375 218**
**A2**

## (12) EUROPEAN PATENT APPLICATION

(21) Application number: 89312806.6

(22) Date of filing: 08.12.89

(51) Int. Cl.5: **A01H 4/00, A01G 1/02**

(30) Priority: 08.12.88 JP 308747/88
19.06.89 JP 154603/89
19.06.89 JP 154604/89
19.06.89 JP 154605/89

(43) Date of publication of application:
27.06.90 Bulletin 90/26

(84) Designated Contracting States:
AT BE CH DE ES FR GB GR IT LI LU NL SE

(71) Applicant: **MITSUI PETROCHEMICAL INDUSTRIES, LTD.**
2-5, Kasumigaseki 3-chome Chiyoda-ku Tokyo 100(JP)

(72) Inventor: **Hirata, Yukimasa c/o Mitsui Petrochem. Ind. Ltd.**
**1-2 Waki 6-chome Waki-cho**
**Kuga-gun Yamaguchi 740(JP)**
Inventor: **Takahashi, Shigeru c/o Mitsui Petrochem. Ind. Ltd.**
**1-2 Waki 6-chome Waki-cho**
**Kuga-gun Yamaguchi 740(JP)**
Inventor: **Moriwaki, Rie c/o Mitsui Petrochem. Ind. Ltd.**
**1-2 Waki 6-chome Waki-cho**
**Kuga-gun Yamaguchi 740(JP)**
Inventor: **Koyama, Atsuko c/o Mitsui Petrochem. Ind. Ltd.**
**1-2 Waki 6-chome Waki-cho**
**Kuga-gun Yamaguchi 740(JP)**

(74) Representative: **Myerscough, Philip Boyd et al**
**J.A.Kemp & Co. 14, South Square Gray's Inn**
**London, WC1R 5EU(GB)**

(54) Method of multiplying plant belonging to the genus asparagus.

(57) A method is disclosed for multiplying a plant belonging to the genus Asparagus in large quantities, which comprises culturing a tissue or a callus of a plant belonging to the genus Asparagus to form a tuber-like tissue or a crown.

FIG. 1

# METHOD FOR MULTIPLYING PLANT BELONGING TO THE GENUS *ASPARAGUS*

## BACKGROUND OF THE INVENTION

This invention relates to a method for multiplying a plant belonging to the genus *Asparagus*.

Hitherto, the multiplication of a plant belonging to the genus *Asparagus* has been carried out by seeding or division. A plant of the genus *Asparagus* is dioecious, and its male plant is considered to be advantageous in respect of quality and cultivation administration. In addition, this plant is a crop having a large genetic variation, so that the multiplication by a nursery plant has brought serious cultivational problems such as causing the contamination of male plants and causing the differences of yield and quality in every plant. Furthermore, the multiplication by division requires many hands and years, is very inefficient and has a very high risk of communicating viral disease and the like.

For the purpose of improving the above problems, the mass multiplication of an excellent plant clone according to the tissue culture has recently received attention. Generally, the mass multiplication of an asparagus is carried out by firstly culturing a tissue section to multiply a large quantity of shoots and then making the shoots grow into nursery plants by transplanting each shoot into a rooting medium to differenciate adventitious roots. However, this method has a problem that the multiplication efficiency is low because the rooting ratio is generally low. On the other hand, an attempt is being made in order to establish a method using a somatic embryo developed from a callus formed by culturing a tissue section as a mass multiplication technique (e.g., The gist of Announcement of the Horticultural Science Society, 1986 Autumn Seminar, pp. 210~211, held at University of the Ryukyus, November 23~25, 1986; The gist of Announcement of the Horticultural Science Society, pp. 254~255, held at Kyushu University, October 7~9). The growing of a somatic embryo dissolves the problem of low rooting ratio. However, it becomes a problem that most of isolated somatic embryos turn into calluses or cause distortion.

## SUMMARY OF THE INVENTION

Upon recognizing that the conventional tissue culture methods of a plant belonging to the genus *Asparagus* have the aforementioned problems, the present inventors studied a method for efficiently multiplying a nursery plant of a plant belonging to the genus *Asparagus* by a novel tissue culure method different from the conventional ones.

As the result, the present inventors found that the above problems of the prior art could be preferably solved by multiplying a plantlet through a crown. In addition, they found that the problems of the prior art also could be sovled by multiplying a plantlet through a tuber-like tissue or by dividing a stock to be obtained by tissue culture at the time of multiplying a plant belonging to the genus *Asparagus* by tissue culture. On the basis of these findings, they made further progress in their study to find that, in case of carrying out the tissue culture of a plant belonging to the genus *Asparagus* by using a liquid medium having a dissolved oxygen concentration (DO) of 9ppm or more, the propagation of a tissue and a cell or the differenciation of an adventitious root was promoted. Whereby, they completed the present invention.

According to the present invention, a crown, a tuber-like-tissue or a stock can be formed from a plant belonging to the genus *Asparagus* to mass-propagate by tissue culture. In addition, a plantlet and a nursery plant can be efficiently mass-multiplied from the obtained crown, tuber-like tissue or stock.

## BRIEF DESCRIPTION OF THE DRAWINGS

Fig. 1 is a chart showing processes of the formation and the propagation of a crown of a plant belonging to the genus *Asparagus* and the multiplication of a plantlet thereof accroding to the present invention.

Fig. 2 is a photograph showing a crown formed from a section of a stem of asparagus.

Figs. 3 and 4 are photographs showing the state of a crown of asparagus being rooted to regenerate a plantlet.

Fig. 5 is a photograph showing a tissue section of the above crown.

Figs. 6 to 9 show one example of an apparatus which can be used in the present method for multiplying a plant belonging to the genus *Asparagus*.

Fig. 10 shows one example of a method for multiplying ing a plant belonging to the genus *Asparagus* through a tuber-like tissue.

Fig. 11 is a photograph showing a tuber-like tissue of asparagus formed according to the present invention.

Fig. 12 shows one example of a method for multiplying a plant belonging to the genus *Asparagus* by forming a stock.

Fig. 13 is a photograph showing a stock formed and an individual divided from the stock.

Fig. 14 shows one example of an incubator to be used in the method shown in Fig. 12.

DETAILED DESCRIPTION OF THE INVENTION

As previously described, the present invention contempletes providing a method for efficiently mass multiplying a plant belonging to the genus *Asparagus* through the forma tion and mass propagation of a crown, a tuber-like tissue or a stock and the growing of a plantlet thereof by tissue culture.

More specifically, the present invention provedes the following methods:

(1) A method for forming a crown of a plant belonging to the genus *Asparagus* by culturing a tissue or a callus of the plant.

(2) A method for propagating a crown of a plant belonging to the genus *Asparagus* by cutting a section from a crown formed by culturing a tissue or a callus of a plant belonging to the genus *Asparagus*, further culturing the section to form a crown and, at need, repeating the same propagation process of cutting of a crown → culturing of a section → formation of a crown.

(3) A method for multiplying a plantlet of a plant belonging to the genus *Asparagus* by culturing a crown obtained by a method of (1) or (2) to form a plant body.

(4) A method according to (3), in which the step of growing a plantlet includes a step of rooting.

(5) A method for multiplying a nursery plant of a plant belonging to the genus *Asparagus* by growing a plantlet obtained by a method of (3) or (4) into a nursery plant by acclimatization.

(6) A method for forming a tuber-like tissue of a plant belonging to the genus *Asparagus* by culturing a tissue or a callus of the plant.

(7) A method according to (6), in which the tissue of the plant is a tuber-like tissue obtained by culturing a tissue or a callus of a plant belonging to the genus *Asparagus*.

(8) A method for propagating a tuber-like tissue of a plant belonging to the genus *Asparagus* by cutting a section from a tuber-like tissue formed by a method of (6) or (7), further culturing the section to grow a tuber-like tissue and, at need, repeating the same propagation process of cutting of a tuber-like tissue → culturing of a section → enlargement of a tuber-like tissue.

(9) A method for multiplying a plantlet of a plant belonging to the genus *Asparagus* by culturing a tuber-like tissue obtained by a method of (6), (7) or (8) to grow a plantlet.

(10) A method according to (9), in which the step of culturing a tuber-like tissue to grow a plantlet is that of culturing a tuber-like tissue to regenerate the same and culturing the regenerated tuber-like tissue to grow a plantlet.

(11) A method for multiplying a nursery plant of a plant belonging to the genus *Asparagus* by growing a plantlet obtained by a method of (9) or (10) into a nursery plant by acclimatization.

(12) A method for multiplying a plant belonging to the genus *Asparagus* by culturing a tissue or a callus of a plant belonging to the genus *Asparagus* to form a stock of the plant, dividing the obtained stock into individduals each having at least one airial part and root part, culturing the obtained individuals to obtain stocks of the plant and, at need, repeating the same division and propagation process of a stock composed of culturing of the divided individual → forming a stock of the plant.

(13) A method according to (12), in which the step of culuring a tissue or a callus of a plant belonging to the genus *Asparagus* to form a stock of the plant is that composed of culturing a tissue or a callus of a plant belonging to the genus *Asparagus* to form a crown and further culturing the crown to regenerate and root a plantlet to form a stock of the plant.

(14) A method for multiplying a nursery plant of a plant belonging to the genus *Asparagus* by growing a plantlet obtained by a method of (12) or (13) into a nursery plant by acclimatization.

(15) A method of tissue culture of a plant belonging to the genus *Asparagus* by carrying out the tissue culture by using a liquid medium having a dissolved oxygen concentration (DO) of 9ppm or more.

In the aforementioned method (1), it is preferred to use a medium containing 3% or more sucrose, a medium containing $10^{-7}$M to $10^{-4}$M cytokinin or a medium containing $10^{-7}$M to $10^{-4}$M cytokinin and 3% or more sucrose.

In addition, the efficient formation of a superior crown can be attained by carrying out the culture of a tissue or a callus of a plant belonging to the genus *Asparagus* under aeration by using a solid medium, by using a liquid medium impregnated into a supporting material, by intermittently supplying a liquid medium or by spraying a liquid medium.

In the aforementioned method (2), it is preferred to use a medium containing $10^{-8}$M to $10^{-4}$M cytokinin and $10^{-7}$M to $10^{-5}$M auxin.

In the aforementioned method (3), it is preferred to use an antigibberellin or a medium containing $10^{-7}$M to $10^{-5}$M auxin.

In the present invention, all of the plants belonging to the genus *Asparagus* can be used. As a specific example of the plants, *Asparagus officinalis* L. var. *altilis* L. as an edible asparagus can be enumerated. In the present invention, it is preferred to use this species.

Firstly, a method for multiplying a plant belonging to the genus *Asparagus* by culturing a tissue or a callus of a a plant belonging to the genus *Asparagus* to form a crown will be described in detail.

In the present invention, a crown is formed by the tissue culture of a tissue or a callus of a plant belonging to the genus *Asparagus*. As examples of a tissue to be used in this tissue culture, tissues such as an embryo, a shoot apex, a stem, a phyllodium, a subterranean stem, etc. can be enumerated. As a callus in the present invention, the one obtained by culturing the aforementioned tissue, for example, by a publicly known culture method [e.g., *Journal of Horticultural Science Society*, Vol. 40, No. 4, pp. 11~17 (1971)] can be used.

A medium to be used in the above tissue culture is the one in which inorganic components and carbon sources are contained as essential ingredients and plant hormones, vitamins and, at need, amino acids are added.

As inorganic components of the medium, inorganic salts containing elements such as nitrogen, phosphorous, potassium, sodium, calcium, magnesium, sulfur, iron, manganese, zinc, boron, molybdenum, chlorine, iodine, cabalt, etc. can be enumerated. Specifically, compounds such as potassium nitrate, sodium nitrate, ammonium nitrate, potassium chloride, calcium chloride, potassium hydrogenphosphate, sodium dihydrogenphosphate, magnesium sulfate, magnesium chloride, sodium sulfate, ferrous sulfate, ferric sulfate, manganese sulfate, copper sulfate, sodium molybdenum, molybdenum trioxide, potassium iodide, zinc sulfate, boric acid, cobalt chloride, etc. can be exemplified.

As examples of carbon sources of the medium, carbohydrates such as sucrose, glucose, etc. and their derivatives; organic acids such as fatty acid and the like; primary alcohols such as ethanol and the like; etc. can be enumerated.

As examples of plant hormones of the medium, auxins such as 1-naphthaleneacetic acid (NAA), indole-3-acetic acid (IAA), *p*-chlorophenoxyacetic acid, 2,4-dichlorophenoxyacetic acid (2,4-D), indole-3-butylic acid (IBA) and their derivatives and cytokinins such as 6-benzyladenine (BA), kinetin, zeatin, etc. can be enumerated. In addition, Zip [6-($\gamma,\gamma$-dimethylarylamino)-purine] may be used.

As examples of vitamins of the medium, biotin, thiamine (vitamin $B_1$), pyridoxine (vitamin $B_6$), pyridoxal, pyridoxamine, calcium pantothate, ascorbic acid (vitamin C), inositol, nicotinic acid, nicotinamide, riboflavin (vitamin $B_6$), etc. can be enumerated.

As examples of amino acids of the midium, glycine, alanine, glutamine, cystine, phenylalanine, lysine, etc. can be enumerated.

It is desired that the aforementioned medium to be used in the present method is generally used upon adding thereto approx. $0.1\mu$M to approx. 100mM of the aforementioned inorganic ingredients, approx. 0.01mg/$\ell$ to approx. 150mg/$\ell$ of the aforementioned plant hormones and 0 to approx. 1,000mg/$\ell$ of the aforementioned amino acids.

As specific examples of the aforementioned medium to be used for the tissue culture according to the present method, media adjusted by adding the aforementioned carbon sources plant hormones and, at need, the aforementioned vitamins and amino acids to the conventionally know media used for the plant tissue culture, for example, Murashige-Skoog ('62) medium, Linsmaier-Skoog (RM-1965) medium, White ('63) medium, Gamborg's B-5 medium, Mitsui's M-9 medium, Nitch-Nitch medium, etc. can be enumerated. Among these media, a medium adjusted by using Nitch-Nitch midium, Linsmaier-Skoog medium or Murashige-Skoog medium is particularly perferable. With respect to the composition of the above conventionally known media, there is a description, for example, in "New Plant Tissue Culture" [Takeuchi, Nakajima and Furuya, pp. 386~391, Asakura-shoten (1979)]. The aforementioned medium which can be used in the present method is a liquid medium or a solid medium to which generally 0.1 to 2% gelling agent, for example, agar, gelrite or the like is added.

As a medium to be used for forming a crown in the pre sent method, the aforementioned media can be used. However, in case of using a medium to which sucrose is added to give an initial sugar concentration of 3% or more, the ratio of crown formation can be particularly increased.

In case of using a medium containing $10^{-7}$M to $10^{-4}$M cytokinin or a medium containing $10^{-7}$M to

4

$10^{-4}$ M cytokinin and 3% or more sucrose, the ratio of crown formation can be also increased.

In the present invention, a crown can be formed by culturing a tissue or a callus of a plant belonging to the genus *Asparagus* by using the aforementioned media, a plantlet can grown from the propagated crown at need, and a nursery plant of the plant can be obtained by acclimatizing the aforementioned plantlet. The crown in this case means a storage tissue corresponding to a subterranean stem of the whole plant and morphologically corresponding to a shortend stem and indicates an aggregated or coronary cell mass containig a plumule or a plumule primordium which grows into a plumule obtained by culturing a tissue or a callus.

In the present invention, although the yield of crown to be obtained by the tissue culture is low, a crown can be propagated by cutting crowns alone from the culture obtained by the aforementioned method, transferring them to other media and culturing them to enlarge and grow.

If necessary, the propagation process composed of cut ting of a crown → culturing of a section → enlargement of a crown may be repeated. For the enlargement and the growth of a crown, the same media as used for the aforementioned formation of a crown can be used and a medium containing $10^{-8}$ M to $10^{-4}$ M cytokinin and $10^{-9}$ M to $10^{-5}$ M auxin is preferable. In addition, in case of using a medium containing 3% or more (initial concentration) sucrose as shown in Example 5, particularly high propagation ratio can be attained. In Table 5, the size of the obtained crown is indicated by the propagation ratio of a crown. In this table, large numerical value indicates that the size of crown is large and the number of buds to be grown into shoots is increased.

In the present method, the above enlarged crown is cut into a suitable size at need preferably so as to contain at least one bud and the obtained crown sections are cultured repeatedly in the same way. Whereby, a crown can be mass-propagated.

Then, a proper quantity of crowns were extracted from among crowns propagated as above and cultured in rooting media to root and grow buds on the tip of the crowns into plantlets of proper size.

As a rooting medium, for example, a medium adjusted by adding the aforementioned carbon sources, plant hormones and, at need, the aforementioned vitamins and amino acids to a medium such as Murashige-Skoog ('62) medium, Linsmaier- Skoog (RM-1965) medium, White ('63) medium, Gamborg's B-5 medium, Mitusi's M-9 medium, Nitch-Nitch medium or the like can be used. As specific examples thereof, media shown in Example 7 or 8 can be enumerated. The rooting is particularly promoted in case of adding antigiberrellin to a medium. As the antigiberrellin, ancymidol or uniconazol can be enumerated. In addition, the rooting is further promoted in case of using a medium containing $10^{-8}$ M to $10^{-5}$ M auxin.

The above plant body can be grown into a nursery plant by acclimatization. In the present invention, a nursery plant ofa plant belonging to the genus *Asparagus* can be mass-propagated by repeating the methods described above.

Hereinafter, respective methods of the present invention and a crown, a plantlet, etc. obtained by the methods are described, referring to the attached drawings.

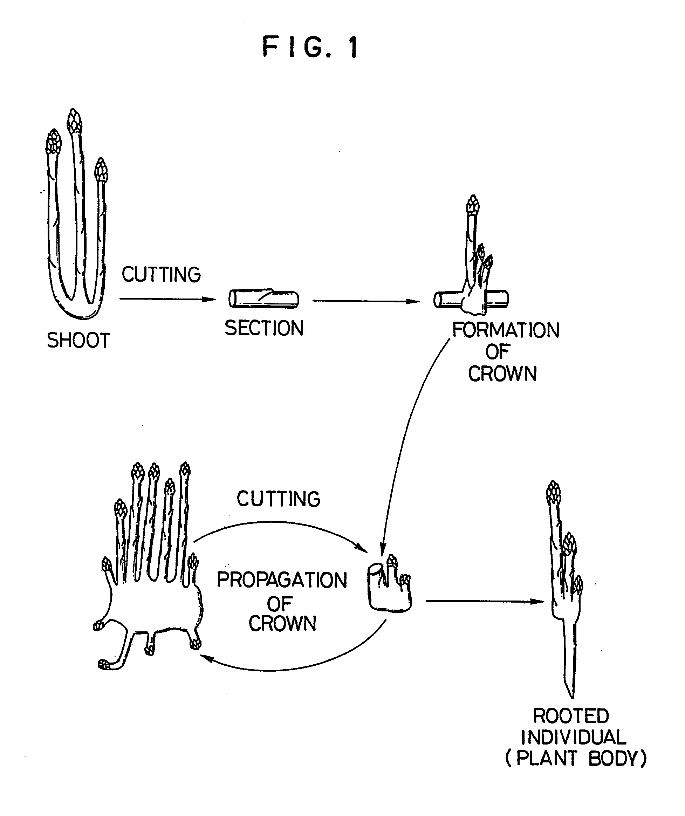

Fig. 1 is a chart showing processes of the formation and the propagation of a crown of a plant belonging to the genus *Asparagus* and the propagation of a plantlet of the plant according to the present methods. In the upper part of the chart, a course of the formation of a crown by culturing a section obtained by cutting a stem of a shoot is shown. On the left side of the lower part of the chart, the propagation cycle in which a crown is propagated from the aforementioned crown is shown. On the right side of the chart, a plantlet rooted and regenerated from thus propa gated crown is shown.

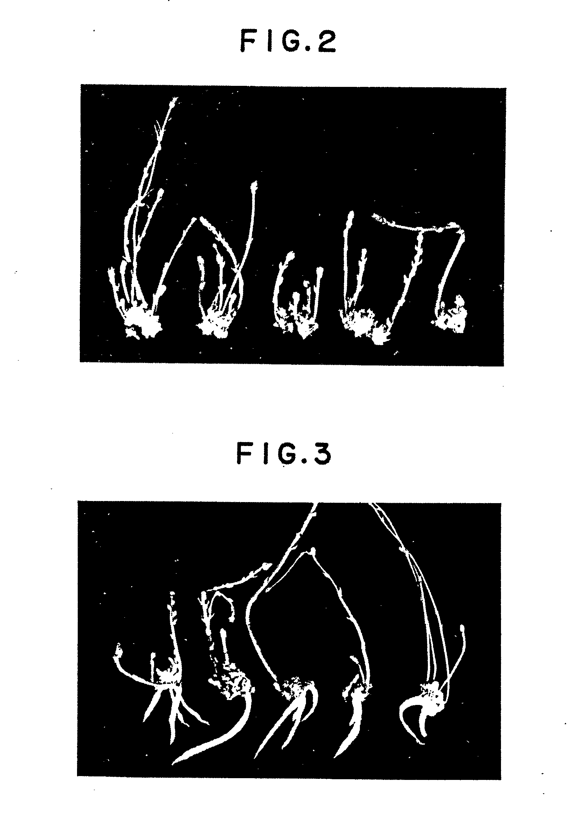

Fig. 2 is a photograph showing a crown formed from a section of a stem of asparagus.

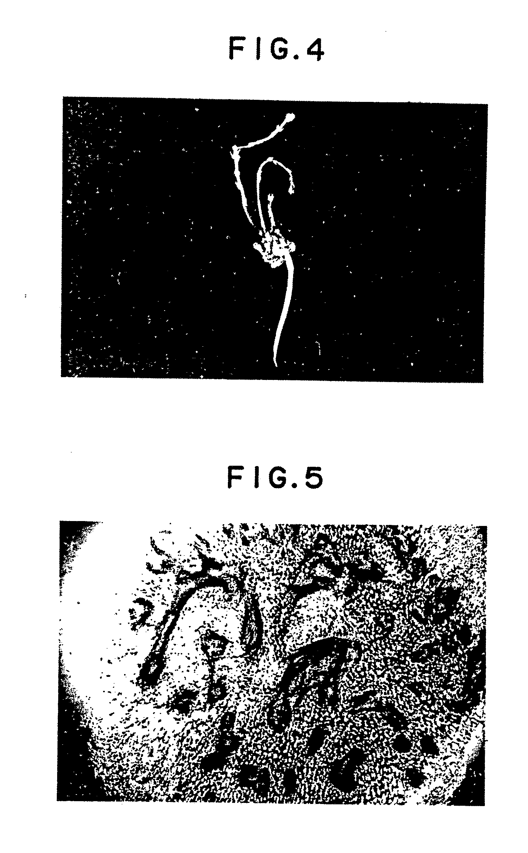

Figs. 3 and 4 are photographs showing the state where a crown of asparagus is rooted to regenerate a plantlet. In Fig. 4, a block in the center is a crown and the finger-like part on its left is a bulblet. The part extended upwards from the crown is a shoot and the part extended downwards from the crown is a root (a white storage root).

Fig. 5 is a photograph showing a tissue section of the above crown, wherein the dark part is a vascular bundle tissue.

According to the present method, not only a crown can be formed from a plant belonging to the genus *Asparagus* but also the crown can be efficiently mass-propagated by the tissue culture. In addition, a plantlet and a nursery plant can be efficiently mass-multiplied through the obtained crown.

In case of multiplying a plant belonging to the genus *Asparagus* by regenerating a plantlet thereof after forming a crown by culturing a tissue or a callus of the plant and propagating the crown at need as described above, more superior nursery plant can be obtained efficiently by carrying out the culture in a process of the formation of a crown, the growing or the propagation of the crown or the growing of the crown into a plantlet including a rooting step according to the following culturing methods (A) to (D):

(A) Carrying out the culture under aeration by using a solid medium.

5

(B) Carrying out the culture under aeration by using a liquid medium impregnated into a supporting material.

(C) Carrying out the culture under aeration by placing a culture material on the supporting material and intermittently making the culture material or at least a part of the culture immersed in a liquid medium by intermittently supplying and exhausting the liquid medium.

(D) Carrying out the culture under aeration by using a liquid medium impregnated into a supporting material and spraying the liquid medium.

Hereinafter, the culture method of the above (A) will be proved.

A solid medium to be used in this method contains the same constituents as the aforementioned medium for the crown formation, the crown propagation or the plantlet growing and is adjusted by adding thereto generally 0.1 to 2% gelling agent such as agar, gelrite or the like.

As methods of aeration, forced aeration using an air pump, a gas bomb, a fan, etc. and natural aeration such as giving an airing to an incubator with a filter, utilizing the expansion and the shrinkage of air according to the temprature change, etc. can be enumerated. As gas to be used, air, or a gas prepared by mixing carbon dioxide, nitrogen, etc. into oxygen can be used. In this case, a mixed gas containing 5 to 20vol% oxygen and 400 to 1,000ppm carbon dioxide is particularly desirable. Although the aeration speed depends upon the shape and the size of a culture vessel, the time of aeration of the gas-phase in the culture vessel is desirably 1 to 20 times/hour.

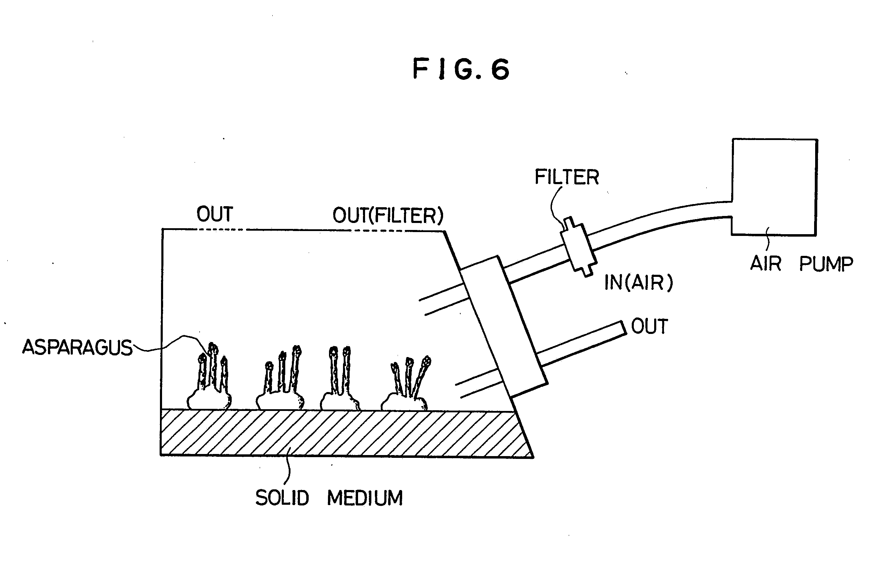

As a culture apparatus, there is no restriction therto as far as it is an apparatus so constructed that a solid medium can be held and the aeration of the gas-phase part can be carried out. Specifically, for example, an apparatus shown in Fig. 6 can be used.

In case of carrying out the culture for the formation of a crown under aeration on a solid medium as described above, a superior crown having a developed vascular bundle system can be formed and the formation speed can be improved. The obtained crown is easy to root and can be free from vitrification. Furthermore, the obtained crown is cut preferably so as to contain one bud. This section is cultured under the same conditions, that is, under aeration by using a solid medium. Whereby, a crown can be enlarged. According to this method, a superior crown which is easy to root and can be free of vitrification can be obtained. At need, the propagation of a crown may be carried out by repeating the propagation process composed of cutting of a crown → tissue culture of a section → enlargement of a crown. Then, steps of rooting the obtained crown and growing of the crown into a plant body is carried out on a solid medium under aeration. Whereby, the rotio of rooting of a storage root and the rooting speed can be increased and the plantlet can be grown into a superior nursery plant.

Hereinafter, the aforementioned culture method (B) will be described.

As a liquid medium to be used in this method, any medium containing the same constituents as that for the aforementioned crown formation, crown propagation or plantlet growing will do. This medium is used by making it impregnated into a supporting material such as wool or its product composed of polymers, e.g., polyester, polyolefin, polyvinyl chloride, polyvinylidene, polyacrylnitrileacetate alcohol, etc.; artificial soil, e.g., rockwool, vermiculite, perlite, etc.; plastic gauze; wire gauze, etc.

As a method of aeration, the same method as described in the aforementioned method (A) can be adopted.

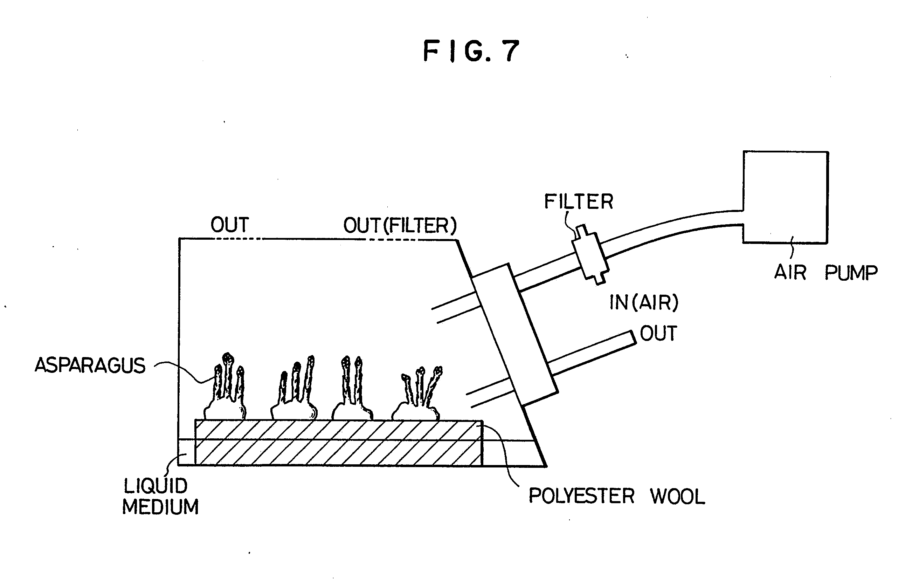

As a culture apparatus to be used for the formation of a crown, there is no restriction thereto as far as it is an apparatus having a supporting material for holding a liquid medium therein and being so constructed that the aeration of the gas-phase part is possible. Specifically, an apparatus shown, for example, in Fig. 7 can be used.

In case of forming a crown according to this method, not only a superior crown having a developed vascular bundle system can be obtained but also the formation speed can be improved. The obtained crown is easy to root and can be free from vitrification. Furthermore, in case of carrying out the culture for enlarging a crown under aeration by using a liquid medium impregnated into a supporting material, a superior crown which is easy to root can be obtained and, in addition, the vitrification can be prevented. In case of carrying out the same liquid culture under aeration also at the time of making the crowns obtained by repeating the propagation at need root and grow into a plantlet, the rooting ratio of a storage root and the rooting speed can be increased and thus a superior seedling can be obtained.

The culture conditions of the method (C) is featured by the intermittent supply and exhaustion of a liquid medium, and a liquid medium and a supporting material and aeration conditions are as same as those of the aforementioned method (B).

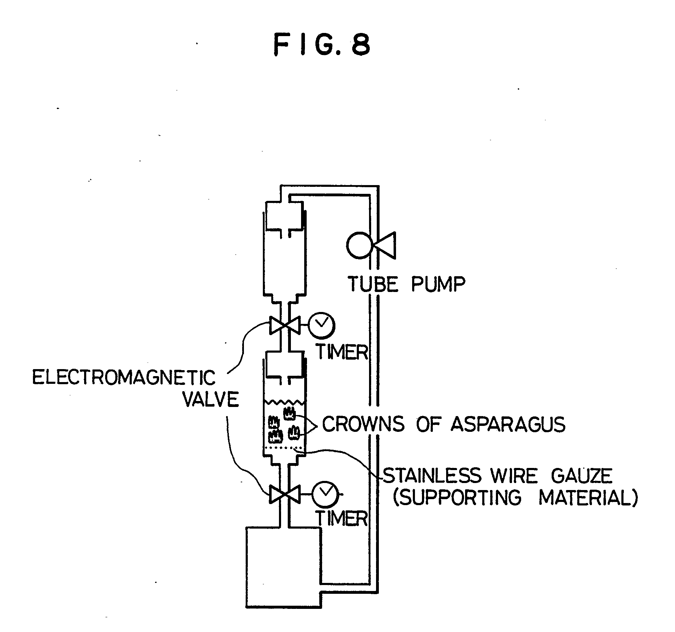

As a culture apparatus to be used in the method (C), there is no restriction thereto as far as it is an apparatus having a supporting material for placing a tissue or a callus and for holding a liquid medium at the time of supplying the liquid medium and being so constructed that the aeration of the gas-phase part and the intermittent supply and exhaustion of the liquid medium are possible. Specifically, an apparatus shown, for example, in Fig. 8 can be used.

In case of forming a crown according to this method, not only a superior crown having a developed vascular bundle system can be obtained but also the formation speed can be improved. The obtained crown is easy to root and can be free from vitrification. In addition, the formation speed of a crown can be incresed to promote the formation of a shoot by intermittently supplying a liquid medium. Furthermore, in case of adopting the method (C) to the culture for enlarging a crown, a superior crown which is easy to root and free from vitrification can be obtained by the aeration and the growth of a crown can be promoted with maintaining the quality thereof by the intermittent supply of a liquid medium. Also in the process of rooting a crown propagated at need and growing into a plantlet, the rooting ratio and the rooting speed of a storage root can be incresed and thus a superior nursery plant can be obtained in case of adopting a liquid medium, the intermittent immersing and the aeration.

Hereinafter, the aforementioned method (D) will be described.

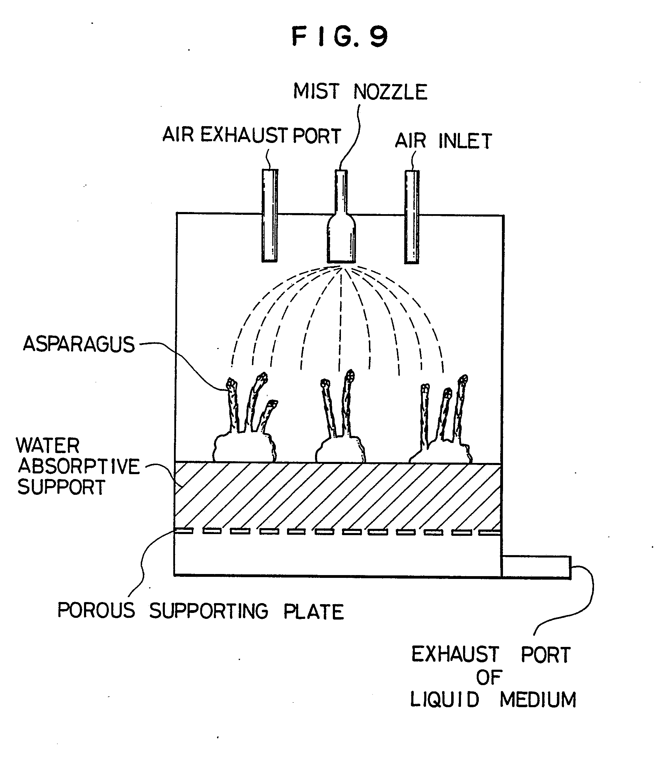

As a liquid medium to be impregnated into a supporting material and the supporting material, the ones as same as those used in the aforementioned method (B) can be used. As aeration conditions, the same conditions as those of the method (B) can be adopted. As a liquid medium to be sprayed, the same liquid medium as that to be impregnated into the supporting material may be used. As examples of a method of spraying, mist spraying, sprinkling, droping, etc. can be enumerated. As a culture apparatus to be used in this method, there is no restriction thereto so far as it is an apparatus having a supporting material to hold a liquid medium and means of carrying out the aeration of the gas-phase part and the spray of the liquid medium and being so constructed that excess liquid medium can be exhausted. For example, a water-absorptive supporting material such as polyester wool or the like is placed on a porous supporting plate such as stainless wire gauze or the like and a liquid medium is impregnated into the water-absorptive supporting material, as shown in Fig. 9. On the upper part of the apparatus, an air inlet and an air outlet are arranged to make the aeration possible and a mist nozzle is provided to spray the liquid medium. Excess liquid medium is dropped from the porrous supporting plate and exhausted from a drain outlet arranged at the lower part of the apparatus.

In case of forming a crown according to this method, not only a superior crown having a developed vascular bundle system can be obtained but also the formation speed can be improved by aeration. The obtained crown is easy to root and can be free from vitrification. In addition, the spray of a liquid medium further increases the formation speed of a crown and can promote the formation of a shoot. In case of adopting this culture method subjected to the liquid medium spraying and the aeration at the time of enlarging and growing the obtained crown by cutting the crown preferably so as to contain at least one bud and culturing the section, a superior crown which is easy to root and free from vitrification can be obtained by aeration and the growth of the crown can be promoted with maintaining the quality thereof by the spray of a liquid medium. In case of adopting this culture method subjected to the liquid culture spraying and the aeration in the process of culturing a crown propagated at need to root and grow into a plantlet, the rooting ratio and the rooting speed of a storage root can be increased.

According to the method for propagating a plant belonging to the genus *Asparagus* by the crown formation as dedcribed above, the plant can be efficiently propagated. In addition, in case of using the aforementioned specific media, adopting any one of the aforementioned culture methods (A) to (D) or combining the aforementioned specific media and any one of the aforementioned culture methods (A) to (D) in the process of the formation of a crown, the en largement of a crown or the regeneration of a plantlet, a superior plantlet or a superior nursery plant of the aforementioned plant can be obtained efficiently in large quantities.

Hereinafter, a method for propagating a plant belonging to the genus *Asparagus* by culturing the tissue or the callus to form a tuber-like tissue will be described in detail.

The plant species to be used are those belonging to the the genus *Asparagus*. This method is particularly suitable for the multiplication of *Asparagus officinalis* as an edible asparagus.

In this method, a tissue or a callus of a plant belonging to the genus *Asparagus* is first cultured to form a tuber-like tissue. As examples of a tissue to be used in the tissue culture in this case, an embryo, a shoot apex, a phyllodium, a subterranean stem, etc. can be enumerated. As a callus, the one obtained by culturing the tissue, for example, according to the publicly known culture method [e.g., *Journal of Horticultural Society*, Vol. 40, No. 4, pp. 11~17 (1971)] can be used.

Here, the tuber-like tissue means a tissue formed by a part of a subterranean stem (crown) being enlarged and grown and storing a reserve substance.

As a tissue for forming this tuber-like tissue, a callus obtained by culturing the above tissue or callus is pre ferable. The crown in this case is a storage tissue corresponding to a subterranean stem of the whole plant and morphologically corresponding to a shortened stem and indicates an aggregated or coronary cell mass containing a plumule or a plumule primordium which grows into a plumule obtained by culturing a tissue or a callus, as described previously.

A medium to be used for the formation of a tuber-like tissue in this method is the one in which inorganic components and carbon sources are contained as essential ingredients and plant hormones, vitamins and, at need, amino acids are added. Specific examples of each components are as same as those described in the method for propagating a plant belonging to the genus *Asparagus* by the crown formation. With respect to specific examples of a preferable medium, it is the same as in the aforementioned propagation method. That is, either a liquid medium or a solid medium generally containing 0.1 to 2% gelling agent such as agar, gelrite, or the like will do.

In the present invention, a plantlet is grown by culturing a tissue or a callus of a plant of a plant belonging to the genus *Asparagus* by using the aforementioned medium to form a tuber-like tissue and subsequently culturing the tuber-like tissue to form a plant body or by culturing a tuber-like tissue propagated by furthere culturing the section obtained by cutting the tuber-like tissue to enlarge the tuber-like tissue and, at need, repeating the same propagation process composed of cutting of a tuber-like tissue → tissue culture of a section → enlargement of a tuber-like tissue.

In the propagation of a tuber-like tissue, the same medium as that used in the aforementioned tuber-like tissue formation is used.

In the above process of growing a plantlet, the plantlet can be regenerated by regenerating a crown from a tuber-like tissue and subsequently culturing this crown.

The above process of growing a plantlet includes the rooting process.

As examples of a medium to be used for growing a plantlet, media adjusted by adding the aforementioned carbon sources, plant hormones and, at need, the aforementioned vitamins and amino acids to media such as Murashige-Skoog ('62) medium, Linsmaier-Skoog (RM-1965) medium, White ('63) medium, Gamborg's B-5 medium, Mitsui's M-9 medium, Nitch-Nitch medium, etc. can be enumerated.

Furthermore, in the present method, a nursery plant of a plant belonging to the genus *Asparagus* can be obtained by acclimatizing the aforementioned plantlet.

Hereinafter, respective methods of the present invention and a tuber-like tissue, a plantlet, etc. obtained by, the methods will be described, referring to the drawings.

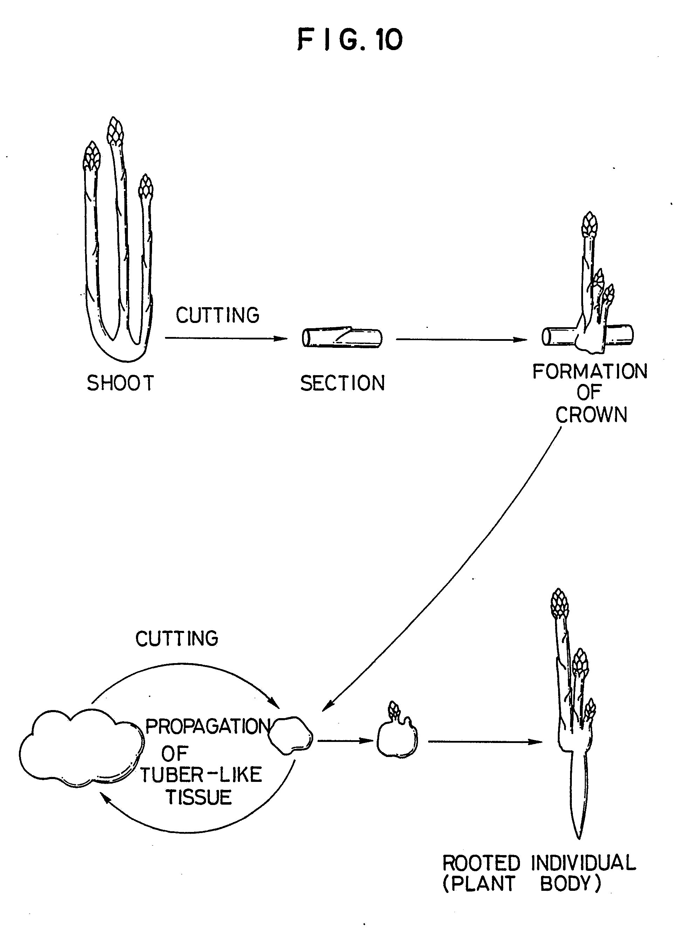

Fig. 10 is a chart showing a process of the formation and the propagation of a tuber-like tissue of a plant belonging to the genus *Asparagus* and the multiplication of a plantlet of the plant according to the present invention. On the upper part of the chart, a course of culturing a section obtained by cutting a stem of a shoot to form a crown is shown. On the left side of the lower part of the chart, the propagation cycle of forming a tuber-like tissue from the aforementioned crown to propagate the tuber-like tissue is shown, and, on the right side of the lower part of the chart, a plantlet rooted and regenerated from thus propagated tuber-like tissue is shown.

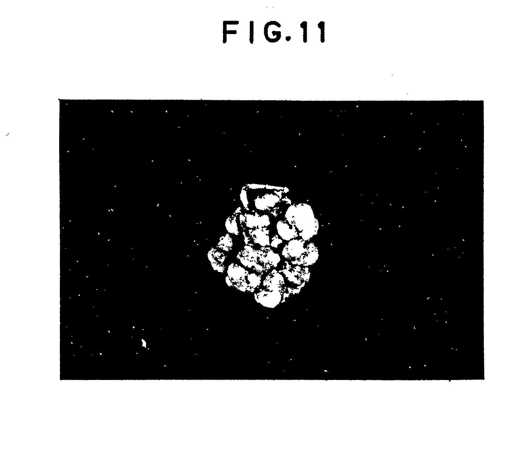

Fig. 11 is a photograph showing a tuber-like tissue obtained in this method.

According to this method, a plantlet or a nursery plant of a plant belonging to the genus *Asparagus* can be efficiently multiplied through a tuber-like tissue.

Hereinafter, a method for efficiently multiplying a plant belonging to the genus *Asparagus* by culturing a tissue or a callus of the plant to form a stock will be described.

In this method, a tissue or a callus of a plant belonging to the genus *Asparagus* is first cultured to form a s stock. As examples of a tissue to be used for the tissue culture in this case, an embryo, a shoot apex, a phyllodium, a subterranean stem, etc. can be enumerated. As a callus, the one obtained by oulturing the tissue, for example, according to the publicly known culture method [e.g., *Journal of Horticultural Society*, Vol. 40, No. 4, pp. 11-17 (1971)] can be used.

Here, the stock means an organ composed of a shoot as an aerial part and roots as a subterranean part being formed on a crown (subterranean stem).

A medium to be used for the formation of a stock in this method is the one in which inorganic components and carbon sources are contained as essential ingredients and plant hormones, vitamins and, at need, amino acids are added. Specific examples of medium components are as same as those used for the aforementioned crown formation. With respect to a preferable medium, either a liquid medium or a solid medium will do.

In the present method, a plant belonging to the genus genus *Asparagus* can be multiplied by culturing a tissue or a callus of a plant belonging to the genus *Asparagus* by using the aforementioned medium to form a stock, dividing the obtained stock into individuals so that each individual contains at least each one aerial part and root part and, at need, repeating the propagation process composed of tissue culture of a divided individual → formation of a stock → division of a stock.

In the tissue culture of a divided individual, the same medium as that used in the aforementioned crown formation can be used.

In the above process of forming a stock by culturing a tissue or a callus of a plant belonging to the genus *Asparagus*, it is preferred that processes of culturing a tissue or a callus of a plant belonging to the genus *Asparagus* to form a crown and culturing the crown to regenerate and root a plantlet are included.

The crown referred in this case means a storage tissue corresponding to a subterranean stem of the whole plant and morphologically corresponding to a shortened stem and indicates an aggregated or coronary cell mass containing a plumule or a plumule primordium which grows into a plumule obtained by culturing a tissue or a callus, as previously described.

In this method, a nursery plant of a plant belonging to the genus *Asparagus* can be multiplied by making a plantlet obtained by the above methods grown into a nursery plant by acclimatization.

Hereinafter, the present method will be described, referring to the attached drawings.

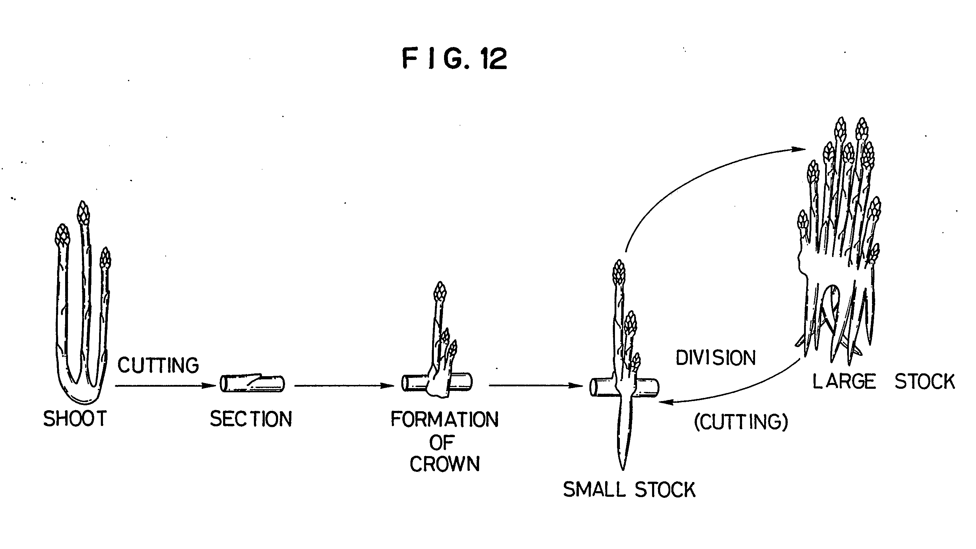

Fig. 12 is one example of the present method and is a chart showing a process of multiplying a plantlet by forming a stock through a crown. In detail, a section obtained by cutting a stem of a shoot is cultured to form a crown. From thus obtained crown, a rooted and regenerated plant body is obtained. This plantlet is enlarged and grown by the tissue culture to form a stock as an aggregate of crowns. Then, this stock is divided into individuals each containing at least each one aerial part and root part.

By repeating the formation and the division of a stock at need, a plantlet can be multiplied.

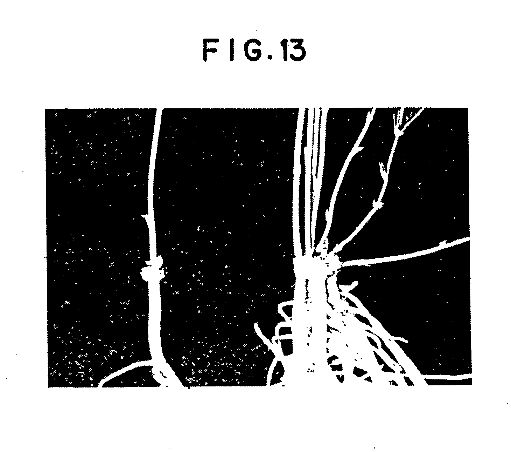

Fig. 13 is a photograph showing a large stock having many shoots and root parts of an asparagus of the present invention before the division (right side of the photograph) and a small stock obtained by dividing the large stock (left side).

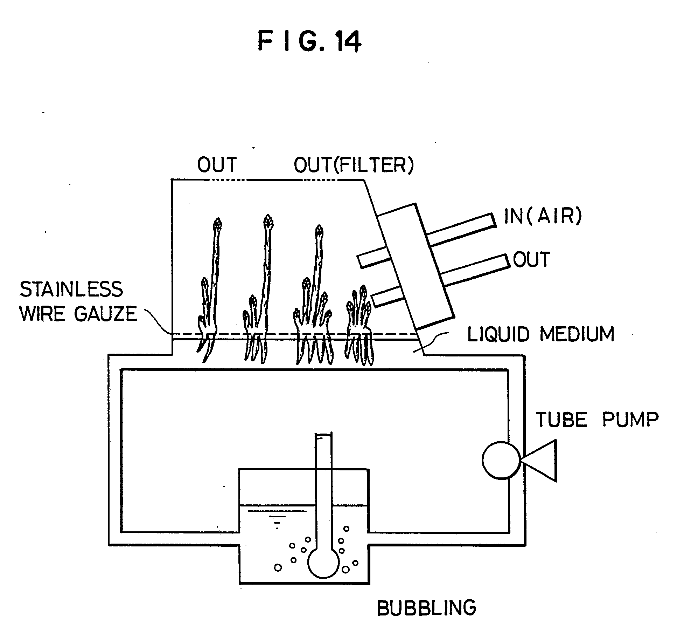

One example of a culture apparatus to be used for the multiplying a plant belonging to the genus *Asparagus* according to this method is shown in Fig. 14. The culture box is composed of 2 containers. One container is for culturing a plantlet by using a support made of stainless wire gauze under aeration and the other for containing a liquid medium whose dissolved oxygen concentration is adjusted by bubbling and enabling to circulate the liquid medium to the former container.

According to this method, a plantlet or a nursery plant of a plant belonging to the genus *Asparagus* can be multiplied efficiently.

Hereinafter, a method for culturing a tissue or a callus of a plant belonging to the genus *Asparagus* by using a liquid medium having a dissolved oxygen concentration (DO) of 9ppm or more will be described.

A plant to which the present tissue culture method is applicable is a plant belonging to the genus *Asparagus*. As specific examples thereof, *Asparagus officinalis* L. var. *altilis* L. and the like can be enumerated.

In the present tissue culture method, the tissue culture is carried out by using a tissue or a callus of the above plant belonging to the genus *Asparagus*. As specific examples of the tissue, an embryo, a shoot apex, a stem, a phyllodium, a subterranean stem, etc. can be enumerated. As the callus, a callus obtained by culturing the above tissues according to the publicly known method can be used.

This method is featured by carrying out the tissue culture by using a liquid medium having a dissolved oxygen concentration (DO) of 9ppm or more, preferably 9 to 20ppm. In order to make the DO 9ppm or more, it is preferable to air a gas having an oxygen concentration higher than common air in the liquid medium. As the gas containing oxygen, oxygen may be used independently or a gas prepared by mixing gases such as air, nitrogen, carbon dioxide, etc. into oxygen may be used. The oxygen content of this mixed gas is generally 22 to 100 vol%, preferably 25 to 100 vol%. The aertion speed of oxygen or the mixed gass into a liquid medium depends upon the shape of a culture apparatus. However, an aeration speed on the order of 0.1 to 30 on the oxygen transfer capacity constant (KLa) basis is generally preferred.

A medium to be used in this method is the one in which inorganic components and carbon sources are contained as essential ingredients and plant hormones, vitamins and, at need, amino acids are added. Specific examples of each components are as same as those mentioned in the aforementioned crown formation.

The present invention is applicable in case of culturing a tissue or a callus of a plant belonging to the genus *Asparagus*. It is particularly useful if it is applied to the tissue culture for propagating the plant through the aforementioned crown, tuber-like tissue or a stock.

In case of adopting a tissue culture method using a liquid medium having a DO (dissolved oxygen concentration) of 9ppm or more, the differentiation, propagation and growth of a tissue or a cell of a plant belonging to the genus *Asparagus* are strikingly promoted to enable the mass-multiplication of a nursery plant through the tissue or the cell. Therefore, according to the present invenion, a plantlet of high quality can be mass-cultured efficiently from a tissue or a callus of a plant belonging to the genus *Asparagus* as compared with the conventional methods and thus a nursery plant can be mass-multiplied.

Hereinafter, the present methods will be described specifically, referring to examples.

Example 1 Formation of Crown

A shoot of an asparagus (cultivar: Mary Washington 500) which had been maintained in sterile line was so cut into sections as materials that each section have a length of 7 to 10cm including a knot. A medium was prepared by using inorganic components of the MS medium and organic components of the Nitch-Nitch medium as basic ingredients, except that the concentrations of $NH_4NO_3$ and $CaC\ell_2$ were respectively adjusted to 1/2-fold and 2-fold and $10^{-6}$M glutamin was added. This medium was used as a basal medium (hereinafter abbreviated to "ASP medium").

Then, 10, 30 and 60g/$\ell$ of sucrose (concentration: 1, 3 and 6%) were added to this medium, followed by the adjustment of pH to 5.8 and the addition of $10^{-7}$M IBA as auxin, $10^{-6}$M BA as cytokinin and $10^{-6}$M ancymidol as antigibberellin. After dispensing thus adjusted medium in 30m$\ell$ portions into commercially available glass bottle (volume: approx. 220m$\ell$), 100 sections of the asparagus stem were placed there on and cultured for 9 weeks at 25°Cunder 3,000 lux. The results were given in Table 1.

## T a b l e  1

| Sucrose Concentration | Ratio of Crown Formation |
|---|---|
| 1% | 5.0% |
| 3% | 55.0% |
| 6% | 62.0% |

$$\text{Ratio of Crown Formation } (\%) = \frac{\text{Number of Sections which Formed Crowns}}{\text{Number of Sections Tested}} \times 100$$

Example 2 Formation of Crown

A shoot of an asparagus (cultivar: Hokkai 100) which had been maintained in sterile line was so cut into sections as materials that each section could have a length of 7 to 10cm including a knot. A medium was prepared by using inorganic components of the MS medium and organic components of the Nitch-Nitch medium as basic ingredients, except that the concentrations of $NH_4NO_3$ and $CaC\ell_2$ were respectively adjusted to 1/2-fold and 2-fold and $10^{-6}$M glutamin was added. This medium was used as a basal medium.

Then, 30 and 60g/$\ell$ of sucrose and 7g/$\ell$ of agar were added to this medium, followed by the adjustment of pH to 5.8 and the addition of $10^{-7}$M 3-indolebbutyric acid (IBA) as auxin, $3 \times 10^{-7}$M or $10^{-6}$M benzyladenine (BA) as cytokinin and no or $10^{-6}$M ancymidol as antigibberellin. After dispensing thus adjusted medium in 30m$\ell$ portions into commercially available glass bottles (volume: approx. 220m$\ell$), 100 sections of the asparagus stem were placed thereon and cultured for 9 weeks at 25°C under 3,000 lux. The results were given in Table 1.

Table 2

|  |  |  | (9-week culture) |
| --- | --- | --- | --- |
| Cytokinin | Sucrose | Ancymidol | Ratio of Crown Formation |
| BA $3 \times 10^{-7}$M | 3% | None | 10.0% |
|  | 6% | None | 18.0% |
|  | 3% | $10^{-6}$M | 12.7% |
| BA $10^{-6}$M | 3% | None | 26.7% |
|  | 6% | None | 45.3% |
|  | 3% | $10^{-6}$M | 57.3% |

Example 3 Formation of Crown

A shoot of an asparagus (cultivar: Hokkai 100) which had been maintained in sterile line was so cut into sections as materials that each section could have a length of 7 to 10cm including a knot. As a medium, a liquid medium of pH 5.8 using the ASP medium as the basal medium and containing 0, 10, 20, 30, 40, 50, 60, 70, 80 and 90g/ℓ of sucrose was used, to which $10^{-6}$M IBA as auxin, $3 \times 10^{-6}$M BA as cytokinin and $10^{-6}$M ancymidol as antigibberellin. After dispensing thus adjusted medium in 40mℓ portions into commercially available glass bottles (volume: approx. 220mℓ) together with each approx. 0.9g of polyester wool, 100 sections of the above asparagus stem were placed thereon and cultured for 4 weeks at 25°C under 3,000 lux. The results were given in Table 3.

Table 3

|  |  | (4-week culture) |
| --- | --- | --- |
| Sucrose | (Conc.) | Ratio of Crown Formation |
| 0g/ℓ | (0%) | 0.0% |
| 10g/ℓ | (1%) | 22.3% |
| 20g/ℓ | (2%) | 45.2% |
| 30g/ℓ | (3%) | 61.6% |
| 40g/ℓ | (4%) | 88.9% |
| 50g/ℓ | (5%) | 78.6% |
| 60g/ℓ | (6%) | 81.8% |
| 70/gℓ | (7%) | 78.0% |
| 80/gℓ | (8%) | 85.4% |
| 90/gℓ | (9%) | 78.6% |
| IBA $10^{-6}$M, BA $3 \times 10^{-6}$M, ancymidol $10^{-6}$M | | |

Example 4 Formation of Crown

A shoot of an asparagus (cultivar: Hokkai 100) which had been maintained in sterile line was so cut into sections as materials that each section could have a length of 7 to 10cm including a knot. As a medium, a liquid medium of pH 5.8 using the ASP medium as the basal medium and containing 60g/ℓ of sucrose was used. To this medium, were added $3 \times 10^{-7}$M IBA as auxin, $10^{-5}$M, $3 \times 10^{-6}$M, $10^{-6}$M and $3 \times 10^{-7}$ and 0M BA as cytokinin and $10^{-6}$M ancymidol as antigibberellin. After dispensing thus adjusted medium in 40mℓ

11

portions into commercially available glass bottles (volume: approx. 220mℓ) together with approx. 0.9g of polyester wool, 100 sections of the above asparagus stem were placed thereon and cultured for 4 weeks at 25°C under 3,000 lux. The results were given in Table 4.

Comparative Example 1

Sections of an asparagus stem were cultured in the same manner as in Example 4, except that BA was not added. The results were given in Table 4.

Table 4

| | | (4-week culture) | |
|---|---|---|---|
| | | Cytokinin | Ratio of Crown Formation |
| Example 4 | | $10^{-5}$ M | 47% |
| | | $3 \times 10^{-6}$ M | 49% |
| | | $10^{-6}$ M | 18% |
| Comparative Example 1 | | OM | 0% |
| IBA $3 \times 10^{-7}$ M, ancymidol $10^{-6}$ M | | | |

Example 5  Propagation of Crown

A crown formed on the basal part of an asparagus (cultivar: Hokkai 100) which had been maintained in sterile line was so cut into sections as materials that each section could contain 2 or 3 buds. The medium was prepared by using the ASP medium as the basis, adding 10, 30 and 60g/ℓ of sucrose and 7g/ℓ of agar and adjusting the pH to 5.7. To this medium, were added $10^{-7}$ M IBA as auxin, $10^{-6}$ M BA as cytokinin and $10^{-5}$ M ancymidol as antigibberellin. After dispensing thus adjusted medium in 40mℓ portions into commercially available glass bottles (volume: approx. 220mℓ), each 5 sections of the above asparagus crown were placed thereon (10 glass bottles/plot) and cultured for 4 weeks at 25°C under 3,000 lux. The results were given in Table 5.

Table 5

| Sucrose Concentration | Ratio of Crown Propagation |
|---|---|
| 10g/ℓ (1%) | 1.4 |
| 30g/ℓ (3%) | 3.2 |
| 60g/ℓ (6%) | 4.8 |

$$\text{Ratio of Crown Propagation (times)} = \frac{\text{Number of Bud Observed with the Naked Eye at the End of Culture}}{\text{Number of Bud Observed with the Naked Eye at the Start of Culture}}$$

12

Example 6 Propagation of Crown

A crown formed on the basal part of a rooted seedling of an asparagus (cultivar: Hokkai 100) which had been maintained in sterile line was so cut into sections as materials that each section could contain one bud. Then, a medium was prepared by using the ASP medium as the basal medium, adding 60g/$\ell$ of sucrose and 7g/$\ell$ of agar and adjusting the pH to 5.7. To thus adjusted medium, were added $3 \times 10^{-8}$, $10^{-7}$M and $3 \times 10^{-7}$M NAA or $3 \times 10^{-8}$M, $10^{-7}$M and $3 \times 10^{-7}$M IBA as auxin, $10^{-6}$M BA as cytokinin and $10^{-5}$M ancymidol as antigibberellin. After dispensing thus adjusted medium in 30m$\ell$ portions into commercially available glass bottles (volume: approx. 200m$\ell$), 5 sections of the above asparagus crown were placed thereon (10 glass bottles/plot) and cultured for 4 weeks at 25°C under 3,000 lux. The results were given in Table 6.

Table 6

| | | (4-week culture) |
|---|---|---|
| Cytokinin | Auxin | Ratio of Crown Propagation |
| BA $10^{-6}$M | NAA $3 \times 10^{-8}$M | 3.6 |
| $10^{-6}$M | $10^{-6}$M | 3.2 |
| $10^{-6}$M | $3 \times 10^{-7}$M | 2.2 |
| $10^{-6}$M | IBA $3 \times 10^{-8}$M | 2.8 |
| $10^{-6}$M | $10^{-7}$M | 4.8 |
| $10^{-6}$M | $3 \times 10^{-7}$M | 4.4 |
| ancymidol $10^{-6}$M | | |

Example 7 Growth into Plantlet

A crown of an asparagus (cultivar: Hokkai 100) which had been maintained by the technique of Example 5 was so cut into sections as materials that each section could contain 2 or 3 buds. A medium was prepared by using the ASP medium as the basis, adding 10, 30 and 60g/$\ell$ of sucrose and adjusting the pH to 5.8. To thus adjusted medium, were added $3 \times 10^{-6}$M NAA as auxin and $3 \times 10^{-6}$M ancymidol as antigibberellin. After dispensing thus prepared medium in 30m$\ell$ portions into comercially available glass bottles (volume: approx. 220m$\ell$), each 5 sections of the above asparagus crown were placed thereon (10 glass bottles/plot) and cultured for 4 weeks at 25°C under 3,000 lux. Whereby, rooted plantlets were obtained. The results on the rooting ratio were given in Table 7.

T a b l e   7

| Sucrose (%) | Rooting Ratio (%) |
|:-----------:|:-----------------:|
| 1 | 20.0% |
| 3 | 53.0% |
| 6 | 40.0% |

NAA $3\times10^{-6}$M, ancymidol $3\times10^{-6}$M

$$\text{Rooting Ratio (\%)} = \frac{\text{Number of Crown which Formed White Storage Root}}{\text{Number of Crown Tested}} \times 100$$

Example 8 Propagation of Crown

A crown formed on the basal part of a rooted nursery plant of an asparagus (cultivar: Hokkai 100) which had been maintained by the technique of Example 5 was so cut into sections as materials that each section could contain one bud. Then, a medium was prepared by using the ASP medium as the basal medium, adding 30g/ℓ of sucrose and 7g/ℓ of agar and adjusting the pH to 5.7. To thus adjusted medium, were added $3\times10^{-6}$M NAA as auxin and no or $3\times10^{-6}$M ancymidol as antigibberellin. After dispensing thus adjusted medium in 30mℓ portions into commercially available glass bottles (volume: approx. 200mℓ), each 5 sections of the above asparagus crown were placed thereon (10 glass bottles/plot) and cultured for 4 weeks at 25°C under 3,000 lux. Whereby, rooted individuals were obtained. The results on the rooting ratio were given in Table 8.

Table 8

| (4-week culture) | |
|:---:|:---:|
| Ancymidol | Rooting Ratio (%) |
| No addition | 40.0 |
| $3\times10^{-6}$M | 53.0 |
| NAA $3\times10^{-6}$M | |

Example 9 Growth into a Plantlet

A crown of an asparagus (cultivar: Hokkai 100) maintained by the technique of Example 5 was so cut into sections as materials that each section could contain 2 to 3 buds. As a medium, a liquid medium of pH 5.8 using the ASP medium as the basis and containing 60g/ℓ of sucrose was used. To thus adjusted medium, were added $3\times10^{-6}$M, $10^{-6}$M, $3\times10^{-7}$M, $10^{-7}$M and $3\times10^{-8}$M and $10^{-8}$M IBA as auxin, $10^{-7}$M BA as cytokinin and $3\times10^{-6}$M ancymidol as antigibberellin. After dispensing thus adjusted medium in 40mℓ portions into commercially available glass bottles (volume: approx. 200mℓ) together with approx. 0.9g of polyester wool, 100 sections of the above asparagus crown were placed thereon and cultured for 4 weeks at 25° C under 3,000 lux. Whereby, rooted individuals were obtained. The results were given in Table 9.

Comparative Example 2

The sections were cultured in the same manner as in Example 9, except that IBA was not added. The results were given in Table 9.

Table 9

| | (4-week culture) | |
|---|---|---|
| | Auxin | Rooting Ratio |
| Example 9 | BA $3\times10^{-6}$ M | 52% |
| | $10^{-6}$ M | 60% |
| | $3\times10^{-7}$ M | 68% |
| | $10^{-7}$ M | 53% |
| | $3\times10^{-8}$ M | 66% |
| | $10^{-8}$ M | 68% |
| Comparative Example 2 | 0M | 36% |
| BA $10^{-7}$ M, ancymidol $10^{-6}$ M | | |

The results given in Tables 1 to 4 show that a crown is formed by the tissue culture of a stem section and that the ratio of crown formation is particularly high in case of adjusting the sucrose concentration to 3% or more.

The results given in Tables 5 and 6 show that a crown is propagated by the tissue culture of the obtained crown and that the ratio of crown propagation is particularly high in case of adjusting the sucrose concentration to 3% or more.

The results given in Table 7 to 9 show that a plant body can be regenerated by carrying out the tissue culture of the obtained crown to root the crown, that is, the ob tained crown can be grown into a nursery plant. In addition, the results show that the rooting ratio is particularly increased in case of carrying out the tissue culture in a medium containing ancymidol as antigibberellin.

Example 10 Culture Method A

A shoot of an asparagus (cultivar: Hokkai 100) which had been maintained in sterile line was so cut into sections as materials that each section could have a length of 7 to 10cm including a knot. The basal medium was prepared by using inorganic components of the MS medium and organic components of the Nitch-Nitch medium as basic ingredients, except that the concentrations of $NH_4NO_3$ and $CaCl_2$ were respectively adjusted to 1/2-fold and 2-fold and $10^{-6}$ M glutamine was added (hereinafter abbreviated to "ASP medium").

As a medium to be used here, a liquid medium of pH 5.8 using the ASP medium as a basis and containing 60g/ℓ of su crose and 2.5g/ℓ of gelrite was used. To thus adjusted medium, were added $10^{-6}$ M IBA as auxin, $3\times10^{-6}$ M BA as cytokinin and $10^{-6}$ M ancymidol as antigibberellin. After dispensing thus adjusted medium in 180mℓ portions into commercially available culture boxes (having a volume of approx. 220mℓ and having a cap equipped with silicon tubes and a gas filtration filter), each 50 sections (200 sections in total) of the above asparagus stem were placed thereon and cultured for 4 weeks at 25° C under 3,000 lux. at an aeration speed of 30mℓ/min. The results were given in Table 10.

Comparative Example 3

The tissue culture was carried out in the same manner as in Example 10, except that the aeration of air was not adopted. The results were given in Table 10.

Example 11 Culture Method A

A crown of an asparagus (cultivar: Hokkai 100) obtained by the technique of Example 10 was so cut into sections as materials that each section could contain 2 to 3 buds. The medium was prepared by using the ASP medium as the basis, adding 60g/ℓ of sucrose and 2.5g/ℓ of gelrite and adjusting the pH to 5.7. To thus adjusted medium, were added $10^{-7}$M IBA as auxin, $10^{-6}$M BA as cytokinin and $10^{-6}$M ancymidol as antigibberellin. After dispensing this medium in 180mℓ portions into commercially available culture boxes (having a volume of approx. 1,000mℓ and its cap being equipped with silicon tubes and a gas filtration filter), 25 sections/box (100 sections in total) of the above asparagus stem were placed thereon and cultured for 4 weeks at 25°C under 3,000 lux. at an aeration speed of 30mℓ/min. The results were given in Table 11.

Comparative Example 4

The tissue culture was carried out in the same manner as in Example 11, except that the aeration of air was not adopted. The results were given in Table 11.

Example 12 Culture Method A

A crown of an asparagus (cultivar: Hokkai 100) maintained by the technique of Example 10 was so cut into sections as materials that each section could contain 2 to 3 buds. The medium was prepared by using the ASP medium as the basis, adding 60g/ℓ of sucrose and 2.5g/ℓ of gelrite and adjusting the pH to 5.7. To thus adjusted medium, were added $10^{-6}$M IBA as auxin, $10^{-7}$M BA as cytokinin and $10^{-6}$M ancymidol as antigibberellin. After dispensing this medium in 180mℓ portions into commercially available culture boxes (having a volume of approx. 1,000mℓ and having a cap equipped with silicon tubes and a gas filtration filter), 25 sections/box (100 sections in total) of the above asparagus stem were placed thereon and cultured for 4 weeks at 25°C under 3,000 lux. at an aeration speed of 30mℓ/min. The results were given in Table 12.

Comparative Example 5

The tissue culture was carried out in the same manner as in Example 12, except that the aeration of air was not adopted. The results were given in Table 11.

Table 10

|  | Ratio of Crown Formation |
|---|---|
| Example 10 | 5.0% |
| Comparative Example 3 | 62.0% |

16

T a b l e   11

|  | Ratio of Crown Propagation |
| --- | --- |
| Example 11 | 3.8 times |
| Comparative Example 4 | 2.8 times |

$$\text{Ratio of Crown Propagation (times)} = \frac{\text{Fresh Weight of Propagated Crowns}}{\text{Fresh Weight of All Tested Crowns}}$$

Table 12

|  | Rooting Ratio | Average Fresh Weight/Rooted Individual |
| --- | --- | --- |
| Example 12 | 70% | 170mg |
| Comparative Example 5 | 28% | 162mg |

Example 13 Culture Method B

A shoot of an asparagus (cultivar: Hokkai 100) which had been maintained in sterile line was so cut into sections as materials that each section could have a length of 7 to 10cm including a knot. The basal medium was prepared by using inorganic components of the MS medium and organic components of the Nitch-Nitch medium, except that the concentrations of $NH_4NO_3$ and $CaCl_2$ were respectively adjusted to 1/2-fold and 2-fold and $10^{-6}$M glutamine was added (hereinafter abbreviated to "ASP medium").

As a medium to be used here, a liquid medium of pH 5.8 using the ASP medium as the basis and containing 60g/$\ell$ of sucrose was used. To thus adjusted medium, were added $10^{-6}$M IBA as auxin, $3 \times 10^{-6}$M BA as cytokinin and $10^{-6}$M ancymidol as antigibberellin. After dispensing this medium in 180m$\ell$ portions into commercially available culture boxes (having a volume of approx. 1,000m$\ell$ and its cap being equipped with silicon tubes and a gas filtration filter) together with approx. 2.0g of polyester wool, 100 sections of the of the above asparagus stem were placed thereon and cultured for 4 weeks at 25°C under 3,000 lux. at an aeration speed of 30m$\ell$/min. The results were given in Table 13.

Comparative Example 6

The tissue culture was carried out in the same manner as in Example 13, except that the aeration of air was not adopted. The results were given in Table 13.

Example 14 Culture Method B

A crown of an asparagus (cultivar: Hokkai 100) obtained by the technique of Example 13 was so cut into sections as materials that each section could contain 2 to 3 buds. The medium was prepared by using the ASP medium as the basis, adding 60g/$\ell$ of sucrose and 2.5g/$\ell$ of gelrite and adjusting the pH to 5.7. To thus adjusted medium, were added $10^{-7}$M IBA as auxin, $10^{-6}$M BA as cytokinin and $10^{-6}$M ancymidol as antigibberellin. After dispensing this medium in 180m$\ell$ portions into commercially available culture boxes (having a volume of approx. 1,000m$\ell$ and its cap being equipped with silicon tubes and a gas filtration filter), 25 sections/box (100 sections in total) of the above asparagus stem were placed thereon and cultured for 4 weeks at 25°C under 3,000 lux. at an aeration speed of 30m$\ell$/min. The results were given in Table 14.

Comparative Example 7

The tissue culture was carried out in the same manner as in Example 14, except that the aeration of air was not adopted. The results were given in Table 14.

Example 15 Culture Method B

A crown of an asparagus (cultivar: Hokkai 100) main tained by the technique of Example 14 was so cut into sections that each section could contain 2 or 3 buds. A medium was prepared by using the ASP medium as the basis, adding 60g/$\ell$ of sucrose and adjusting the pH to 5.7. To thus adjusted medium, were added $10^{-6}$M IBA as auxin, $10^{-7}$M BA as cytokinin and $10^{-6}$M ancymidol as antigibberellin. After dispensing this medium in 180g portions into commercially available culture box (having a volume of approx. 1,000m$\ell$ and its cap being equipped with a silicon tube and a gas filtration filter) together with approx. 3.0g of polyester wool, 25 sections/box (100 sections in total) were placed thereon and cultured for 4 weeks at 25°C under 3,000 lux at an aeration speed of 30m$\ell$/min. The results were given in Table 15.

Comparative Example 8

The tissue culture was carried out in the same manner as in Example 15, except that the aeration was not adopted. The results were given in Table 15.

Table 13

|  | Ratio of Crown Formation |
|---|---|
| Example 13 | 85% |
| Comparative Example 6 | 62% |

Table 14

|  | Ratio of Crown Propagation |
|---|---|
| Example 14 | 4.3 times |
| Comparative Example 7 | 3.5 times |

$$\text{Ratio of Crown Propagation (times)} = \frac{\text{Fresh Weight of Propagated Crowns}}{\text{Fresh Weight of All Tested Crowns}}$$

Table 15

| | Rooting Ratio | Average Fresh Weight/Rooted Individual |
|---|---|---|
| Example 15 | 76% | 195mg |
| Comparative Example 8 | 34% | 180mg |

Example 16 Culture Method C

A shoot of an asparagus (cultivar: Hokkai 100) which had been maintained in sterile line was so cut into sections as materials that each section could have a length of 7 to 10cm including a knot. The basal medium was prepared by using inorganic components of the MS medium and organic components of the Nitch-Nitch medium, except that the concentrations of $NH_4NO_3$ and $CaCl_2$ were respectively adjusted to 1/2-fold and 2-fold and $10^{-6}$M glutamine was added (hereinafter abbreviated to "ASP medium").

As a medium to be used here, a liquid medium of pH 5.8 using the ASP medium as the basis and containing 60g/$l$ of sucrose was used. To thus adjusted medium, were added $10^{-6}$M IBA as auxin, $3 \times 10^{-6}$M BA as cytokinin and $10^{-6}$M ancymidol as antigibberellin. After dispensing this medium in 180m$l$ portions into culture apparatuses (volume: approx. 300m$l$) in which a stainless wire gauze was laid as a supporting material, each 25 sections (100 sections in total) of the above asparagus stem were placed thereon and cultured for 4 weeks at 25°C under 3,000 lux. at an aeration speed of 30m$l$/min. by repeating a 30-minute immersion in the medium and a 2-hour non-immersion. The results were given in Table 16.

Comparative Example 9

The tissue culture was carried out in the same manner as in Example 13, except that the intermittent immersion or the aeration was not operated and the medium was so circulated that the surface of the medium might just touch the stainless wire gauze. The results were given in Table 16.

Example 17 Culture Method C

A crown of an asparagus (cultivar: Hokkai 100) obtained by the technique of Example 16 was cut into sections as materials so that each section could contain 2 to 3 buds. The medium was prepared by using the ASP medium as the basis, adding 60g/$l$ of sucrose and 2.5g/$l$ of gelrite and adjusting the pH to 5.7. To thus adjusted medium, were added $10^{-6}$M IBA as auxin, $10^{-7}$M BA as cytokinin and $10^{-6}$M·ancymidol as antigibberellin. After dispensing this medium in 200m$l$ portions into culture apparatuses (volume: approx. 300m$l$) in which a stainless wire gauze was laid as a supporting material, each 10 sections (50 sections in total) of the above asparagus crown were placed theron and cultured for 4 weeks at 25°C under 3,000 lux. at an aeration speed of 30m$l$/min. by repeating a 30-minute immersion in the medium and a 2-hour non-immersion. The results were given in Table 17.

Comparative Example 10

The tissue culture was carried out in the same manner as in Example 17, except that the intermittent immersion or the aeration was not operated and the medium was so circulated that the surface of the medium might just touch the stainless wire gauze. The results were given in Table 17.

Example 18 Culture Method C

A crown of an asparagus (cultivar: Hokkai 100) maintained by the technique of Example 17 was so cut into sections that each section could contain 2 to 3 buds. A medium was prepared by using the ASP medium as the basis adding 60g/ℓ of sucrose and adjusting the pH to 5.7. To thus adjusted medium were added $10^{-6}$M IBA as auxin, $10^{-7}$M BA as cytokinin and $10^{-6}$M ancymidol as antigibberellin. After dispensing this medium in 200mℓ portions into culture apparatuses (volume: approx.300mℓ) in which a stainles wire gauze was laid as a supporting material, each 10 sections (50 sections in total) of the above asparagus crown were placed theron and cultured for 4 week at 25°C under 3,000 lux. at an aeration speed of 30mℓ/min. by repeating a 30-minute immersion in the medium and a 2-hour non-immersion. The results were given in Table 18.

Comparative Example 11

The tissue culture was carried out in the same manner as in Example 18, except that the intermittent immersion or the aeration was not operated and the medium was so circulated that the surface of the medium might just touch the stainless wire gauze. The results were given in Table 18.

### T a b l e   16

|  | Ratio of Crown Formation | Vitrification Ratio |
|---|---|---|
| **Example 16** | 100% | 0% |
| **Comparative Example 9** | | |
| No Intemittent Immersion | 80% | 15% |
| No Aeration | 64% | 32% |

$$\text{Vitrification Ratio (\%)} = \frac{\text{Number of Vitrified Sections}}{\text{Number of Tested Sections}}$$

T a b l e   17

|  | Ratio of Crown Propagation | Vitrification Ratio |
|---|---|---|
| Example 17 | 5.4 times | 0% |
| Comparative Example 10 |  |  |
| No Intemittent Immersion | 3.8 times | 35% |
| No Aeration | 3.5 times | 50% |

$$\text{Ratio of Crown Propagation (times)} = \frac{\text{Fresh weight of Propagated Crowns}}{\text{Fresh weight of All Tested Crowns}}$$

$$\text{Vitrification Ratio (\%)} = \frac{\text{Fresh Weight of Vitrified Parts of Cultured Crowns}}{\text{Fresh Weight of Cultured Crowns}}$$

T a b l e   18

|  | Rooting Ratio | Average Fresh Weight/ Rooted Individual | Vitrification Ratio |
|---|---|---|---|
| Example 18 | 70% | 310mg | 0% |
| Comparative Example 11 |  |  |  |
| No Intemittent Immersion | 48% | 205mg | 36% |
| No Aeration | 34% | 190mg | 55% |

$$\text{Vitrification Ratio (\%)} = \frac{\text{Number of Tested Sections on which Formed Crowns were Vitrified in the Course of Rooting}}{\text{Number of Tested Sections}}$$

Example 19 Culture Method D

A shoot of an asparagus (cultivar: Hokkai 100) which had been maintained in sterile line was so cut into sections as materials that each section could have a length of 7 to 10cm including a knot. The basal medium was prepared by using inorganic components of the MS medium and organic components of the Nitch-Nitch medium as basic ingredients, except that the concentrations of $NH_4NO_3$ and $CaCl_2$ were

21

respectively adjusted to 1/2-fold and 2-fold and $10^{-6}$M glutamine was added (hereinafter abbreviated to "ASP medium").

As a medium to be used here, a liquid medium of pH 5.8 using the ASP medium as a basis and containing 60g/$\ell$ of sucrose was used. To this medium, were added $10^{-6}$M IBA as auxin, $3 \times 10^{-6}$M BA as cytokinin and $10^{-6}$M ancymidol as antigiberrellin. After dispensing thus adjusted medium in 180m$\ell$ portions into commercially available culture boxes (having a volume of approx. 220m$\ell$ and so improved that the aeration and the mist spray could be operated), each 50 sections (100 sections in total) of the above asparagus stem were placed thereon and cultured for 4 weeks at 25°C under 3,000 lux. at an aeration speed of 30m$\ell$/min. by operating a mist spray at the ratio of 30m$\ell$/time for 5 minuites at 2-hour intervals. The results were given in Table 19.

Comparative Example 12

The tissue culture was carried out in the same manner as in Example 19, except that mist spray or the aeration of air was not adopted. The results were given in Table 19.

Example 20 Culture Method D

A crown of an asparagus (cultivar: Hokkai 100) obtained by the technique of Example 90 was so cut into sections as materials that each section could contain 2 or 3 buds. The medium was prepared by using the ASP medium as the basis, adding 60g/$\ell$ of sucrose and adjusting the pH to 5.7. To thus adjusted medium, were added $10^{-7}$M IBA as auxin. $10^{-6}$M BA as cytokinin and $10^{-6}$M ancymidol as antigibberellin. After dispensing this medium in 180m$\ell$ portions into commercially available culture boxes (having a volume of approx. 1,000m$\ell$ and so improved that the aeration and the mist spary could be operated) together with approx. 2.0g of polyester wool, each 25 sections/box (100 sections in total) of the above asparagus stem were placed thereon and cultured for 4 weeks at 25°C under 3,000 lux. at an aeration speed of 30m$\ell$/min. by opperating the mist spray at the ratio of 30m/time for 5 minutes at 2-hour intervals. The results were given in Table 20.

Comparative Example 13

The tissue culture was carried out in the same manner as in Example 20, except that the mist spray or the aeration was not adopted. The results were given in Table 20.

Example 21 Culture Method D

A crown of an asparagus (cultivar: Hokkai 100) maintained by the technique of Example 20 was so cut into sections as materials that each section could contain 2 or 3 buds. The medium was prepared by using the ASP medium as the basis, adding 60g/$\ell$ of sucrose and adjusting the pH to 5.7. To thus adjusted medium, were added $10^{-7}$M IBA as auxin, $10^{-6}$M BA as cytokinin and $10^{-6}$M ancymidol as antigibberellin. After dispensing this medium in 180m$\ell$ portions into commercially available culture boxes (having a volume of approx. 1,000m$\ell$ and so improved that the aeration and the mist spray could be operated, 50 sections/box of the above asparagus crown were placed thereon and cultured for 4 weeks at 25°C under 3,000 lux. at an aeration speed of 30m$\ell$/min. Whereby,the sections were rooted to give plantlets. The results were given in Table 21.

Comparative Example 14

The tissue culture was carried out in the same manner as in Example 21, except that the mist spray or the aeration was not adopted. The results were given in Table 21.

Table 19

|  | Ratio of Crown Formation | Vitrification Ratio |
|---|---|---|
| Example 19 | 98% | 2% |
| Comparative Example 12 |  |  |
| No Mist Spray | 95% | 5% |
| No Aeration | 72% | 28% |

$$\text{Vitrification Ratio (\%)} = \frac{\text{Number of Vitrified Sections}}{\text{Number of Tested Sections}}$$

Table 20

|  | Ratio of Crown Propagation | Vitrification Ratio |
|---|---|---|
| Example 20 | 5.0 times | 2% |
| Comparative Example 13 |  |  |
| No Mist spray | 4.3 times | 15% |
| No Aeration | 3.5 times | 30% |

$$\text{Ratio of Crown Propagation (times)} = \frac{\text{Fresh weight of Propagated Crowns}}{\text{Fresh weight of All Tested Crowns}}$$

$$\text{Vitrification Ratio (\%)} = \frac{\text{Fresh Weight of Vitrified Crowns}}{\text{Fresh Weight of Obtained Crowns}}$$

T a b l e   21

|  |  | Rooting Ratio | Average Fresh weight/ Rooted Individual | Vitrifi- cation Ratio |
|---|---|---|---|---|
| Example 21 |  | 80% | 270mg | 5% |
| Comparative Example 14 |  |  |  |  |
|  | No Mist Spray | 76% | 200mg | 12% |
|  | No Aeration | 24% | 145mg | 45% |

$$\text{Vitrification Ratio (\%)} = \frac{\text{Number of Vitrified Crowns}}{\text{Number of Tested Crowns}}$$

Example 22 Formation of Tuber-like Tissue

A crown formed on the basal part of an asparagus (cultivar: Hokkai 100) which had been maintained in sterile line was so cut into sections as a material that each section could contain 2 or 3 buds. The basal medium was prepared by using inorganic components of the MS medium and organic components of the Nitch-Nitch medium as basic ingredients, except that the concentrations of $NH_4NO_3$ and $CaCl_2$ were respectively adjusted to 1/2-fold and 2-fold and $10^{-6}M$ glutamin was added (hereinafter abbreviated to "ASP medium").

As a medium to be used here was prepared by using the ASP medium, adding 60g/ℓ of sucrose and 2.5g/ℓ of gelrite and adjusting the pH to 5.7. To this medium, were added $3 \times 10^{-7}M$ IBA as auxin, $10^{-6}M$, $3 \times 10^{-6}M$ and $10^{-5}M$ BA as cytokinin and $10^{-5}M$ ancymidol aa antigiberrellin. After dispensing thus adjusted medium in 30mℓ portions into 10 commercially available glass bottles (volume: 220mℓ), each 5 sections of the above asparagus crown were placed thereon. These 10 bottles were cultured for 8 weeks at 25°C under 3,000 lux. The results were given in Table 22.

Example 23 Propagation of Tuber-like Tissue

A tuber-like tissue of an asparagus (cultivar: Hokkai 100) prepared by the technique of Example 22 was so cut into sections as materials that each section could contain 2 or 3 buds. The medium was prepared by using the ASP medium as the basis, adding 60g/ℓ of sucrose and 7g/ℓ of gelrite and adjusting the pH to 5.7. To thus adjusted medium, was added $3 \times 10^{-6}M$ ancymidol as antigibberellin. After dispensing this medium in 30mℓ portions into 10 commercially available glass bottles (volume: 200mℓ), each 5 sections of the above asparagus tuber-like tissue were placed thereon. These 10 bottles were cultured for 8 weeks at 25°C under 3,000 lux. The results were given in Table 23.

Example 24 Regeneration of Plantlet from Tuber-like Tissue

A tuber-like tissue of an asparagus (cultivar: Hokkai 100) maintained by the technique of Example 20 was so cut into sections as materials that each section could contain 2 or 3 buds. The medium was prepared by using the ASP medium as the basis, adding 10, 30 and 60g/ℓ of sucrose and adjusting the pH to 5.8. To thus adjusted medium, was added $10^{-5}M$ ancymidol as antigibberellin. After dispensing this

24

medium in 40mℓ portions into 10 commercially available glass bottles (having a volume of approx. 220mℓ and using a 10-mm diameter fluorocarbon membrane filter as a cap) together with approx. 0.9g of polyester wool, each 5 sections of the above asparagus tuber-like tissue were placed thereon. These 10 bottles were cultured for 8 weeks at 25°C under 3,000 lux. Whereby, rooted plantlets were regenerated. The results were given in Table 24.

Table 22

| BA Concentration | Ratio of Tuber-like Tissue Formation |
|---|---|
| $10^{-6}$M | 26% |
| $3 \times 10^{-6}$M | 50% |
| $10^{-5}$M | 45% |

$$\text{Ratio of Tuber-like Tissue Formation} \ (\%) = \frac{\text{Number of Crowns which Formed Tuber-like Tissue}}{\text{Number of Tested Crowns}} \times 100$$

Table 23

| Propagation Ratio |
|---|
| 4.5 times |

$$\text{Propagation Ratio (times)} = \frac{\text{Fresh Weight of Propagated Tuber-like Tissues}}{\text{Fresh Weight of Tested Tuber-like tissue}}$$

25

Table 24

| Sucrose Concentration | Rooting Ratio |
|---|---|
| 10g/ℓ | 10% |
| 30g/ℓ | 35% |
| 60gℓ | 44% |

$$\text{Rooting Ratio (\%)} = \frac{\text{Number of Tuber-like Tissues which Formed White Storage Roots}}{\text{Number of Tested Tuber-like Tissue}} \times 100$$

The following Examples 15 to 31 relates to the multiplication of a plant belonging to the genus *Asparagus.*

Example 25

A shoot of an asparagus (cultivar: Hokkai 100) which had been maintained in sterile line was so cut into sections as materials that each section could have a length of 7 to 10 cm including a knot. A medium was prepared by using inorganic components of the MS medium and organic components of the Nitch-Nitch medium as basic ingredients, except that the concentrations of $NH_4NO_3$ and $CaCl_2$ were respectively adjusted to 1/2-fold and 2-fold and $10^{-6}$ M glutamin was added. This medium was used as a basal medium (hereinafter abbreviated to "ASP medium").

As a medium to be used here, a liquid medium of pH 5.8 using the ASP medium as the basis and containing 60g/ℓ of sucrose and 2.5g/ℓ of gelrite was used. To thus adjusted medium, were added $10^{-6}$ M IBA as auxin, $3 \times 10^{-6}$ M BA as cytokinin and $10^{-6}$ M ancymidol as antigibberellin. After dispensing thus adjusted medium in 30mℓ portions into commercially available culture boxes (volume: approx. 1,000mℓ), each 50 sections (200 sections in total) of the above asparagus stem were placed thereon and cultured for 9 weeks at 25°C under 3,000 lux. The results were given in Table 25.

Example 26

A crown of an asparagus (cultivar: Hokkai 100) obtained by the technique of Example 25 was so cut into sections as materials that each section contain 2 or 3 buds. A medium was prepared by using the ASP medium as the basis, adding 60g/ℓ of sucrose and 2.5g/ℓ of gelrite and adjusting the pH to 5.7. To this medium, were added $10^{-7}$ M IBA as auxin, $10^{-6}$ M BA as cytokinin and $10^{-6}$ M ancymidol as antigibberellin. After dispensing thus adjusted medium in 180mℓ portions in commercially available culture boxes (volume: approx. 1,000mℓ), each 25 sections/box (100 sections in total) of the above asparagus crown were placed thereon and cultured for 4 weeks at 25°C under 3,000 lux. The results were given in Table 26.

Example 27

A crown of an asparagus (cultivar: Hokkai 100) maintained by the technique of Example 26 was so cut into sections as materials that each section contain 2 or 3 buds. A medium was prepared by using the ASP medium as the basis, adding 60g/ℓ of sucrose and 2.5g/ℓ of gelrite and adjusting the pH to 5.7. To this medium, were added $10^{-6}$ M IBA as auxin, $10^{-7}$ M BA as cytokinin and $10^{-6}$ M ancymidol as antigibberellin. After dispensing thus adjusted medium in 180mℓ portions in commercially available culture boxes (volume: approx. 1,000mℓ), each 25 sections/box (100 sections in total) of the above asparagus crown were placed thereon and cultured for 4 weeks at 25° C under 3,000 lux. The results were given in Table 27.

Table 25

|  | Ratio of Crown Formation |
|---|---|
| Example 25 | 65% |

T a b l e   26

Ratio of Crown Propagation

Example 26                    3.2 times

$$\text{Ratio of Crown Propagation (times)} = \frac{\text{Fresh Weight of Propagated Crowns}}{\text{Fresh Weight of All Tested Crowns}}$$

T a b l e   27

Rooting Ratio

Example 27                    70%

$$\text{Rooting Ratio (\%)} = \frac{\text{Number of Crowns which Formed White Storage Roots}}{\text{Number of Tested Crowns}} \times 100$$

Example 28

A large stock having plural aerial parts (shoots) and roots parts on a crown maintained by the technique of Example 26 was used as a material. As a medium, a liquid medium of pH 5.8 using the ASP medium as the basis and containing 60g/ℓ of sucrose and 2.5g/ℓ of gelrite was used. To this medium, were added $10^{-7}$M IBA as auxin, $3 \times 10^{-7}$M BA as cytokinin and $10^{-6}$M ancymidol as antigiberellin. After dispensing thus adjusted medium in 30mℓ portions into commercially available glass bottles (volume: approx. 220mℓ), individduals having an aerial part and a root part obtained by dividing the large asparagus stock, that is, 100 divided small asparagus stocks were placed thereon and cultured 6 weeks at 25° C under 3,000 lux. Whereby, these small stocks were enlarged and grown into large stocks having plural shoots and roots. The results obtained by dividing these large stocks were given in Table 28.

Example 29

The culture was carried out in the same manner as in Example 28, except that crowns obtained in Example 28 were used as materials. The results were given in Table 29.

Table 28

| Example 28 | 3.0 times |
|---|---|

$$\text{Propagation Ratio (times)} = \frac{\text{Number of Small Stocks Obtained by Dividing a Large Stock after Tissue Culture}}{\text{Number of Tested Small stocks}}$$

Table 29

| Example 29 | 2.9 times |
|---|---|

Example 30

A rooted individual maintained by the technique of Example 30 was used as material. A medium was prepared by using inorgainc components of the MS medium and organic components of Nitch-Nitch medium as basic ingredients, except that the concentrations of $NH_4NO_3$ and $CaC\ell_2$ were respectively adjusted to 1/2-fold and 2-fold and $10^{-6}M$ glutamin was added. This medium was used as a basal medium (hereinafter abbreviated to "ASP medium").

As a medium to be used here, a liquid medium of pH 5.8 using the ASP medium as the basis and containing 60g/$\ell$ of sucrose was used. To thus adjusted medium, were added $10^{-7}M$ IBA as auxin, $3\times10^{-7}M$ BA as cytokinin and $10^{-6}M$ ancymidol as antigibberellin. After dispensing thus adjusted medium in 500m$\ell$ portions into culture apparatuses shown in Fig. 14 in which stainless wire gauzes were placed, each 20 rooted seedling of the above asparagus were placed thereon and cultrured for 4 weeks at at 25°C under 3,000 lux by adjusting the dissolved oxygen concentration of the liquid medium to approx. 40ppm and circulating the liquid medium at the flow rate of 5m$\ell$/min. The results were given in Table 30.

Example 31

The culture was carried out in the same manner as in Example 30, except that the rooted nursery plant of an asparagus obtained in Example 30 was used as materials.

Table 30

| | Propagation Ratio |
|---|---|
| Example 30 | 4.0 times |

28

Table 31

| | Propagation Ratio |
|---|---|
| Example 31 | 3.8 times |

Example 32

A shoot of an asparagus (cultivar: Hokkai 100) which had been maintained in sterile line was so cut into sections as materials that each section could have a length of 7 to 10cm including a knot. A medium was prepared by using inorgainc components of the MS medium and organic components of Nitch-Nitch medium as basic ingredients, except the concentrations of $NH_4NO_3$ and $CaCl_2$ were respectively adjusted to 1/2-fold and 2-fold and $10^{-6}M$ glutamin was added. This medium was used as a basal medium (hereinafter abbreviated to "ASP medium").

The medium to be used here was prepared by adding 60g/l of sucrose and adjusting the pH to 5.8, followed by addition of $10^{-7}M$ IBA as auxin, $3 \times 10^{-7}M$ BA as cytokinin and $10^{-6}M$ ancymidol as antigibberellin.

After dispensing thus prepared liquid medium in 50ml portions into culture apparatuses (volume: 150ml), each 10 sections/apparatus were planted in the apparatuses. Then, a gas aeration tube equipped with a glass filter was attached to the apparatus. The sections were cultured for 30 days at 25°C under 3,000 lux. under a 16-hour light cycle by blowing pure oxygen passed through a 0.2-μm sterilizing filter at the rate of 3ml/min. through the gas aeration tube [oxygen transfer capacity constant (KLa): 5.0]. The average dissolved oxygen cocentration in the medium during the culture was approx. 40ppm. The results were given in Table 32.

Comparative Example 15

The calture was carried out in the same manner as in Example 32, except that air was aerated instead of pure oxygen. The oxygen transfer capacity constant (KLa) was 5.0 and the average dissolved oxygen concentration in the medium during the culture was 8.1ppm. The results were given in Table 32.

Table 32

| | DO (ppm) | Average Fresh Weight/Individual (mg) |
|---|---|---|
| Example 32 | approx. 40 | 47.3 |
| Comparative Example 15 | 8.1 | 15.6 |

**Claims**

1. A method of multiplying a plant belonging to the genus Asparagus, which comprises culturing a tissue or a callus of a plant belonging to the genus Asparagus to form a tuber-like tissue or a crown.

2. A method according to claim 1, which comprises culturing the tissue or the callus in a medium containing 3% or more sucrose and/or $10^{-7}M$ to $10^{-4}M$ cytokinin.

3. A method according to claim 1 or 2, which comprises culturing the tissue or the callus under aeration using a solid medium or using a liquid medium impregnated into a supporting material.

4. A method according to any one of claims 1 to 3 which comprises placing the tissue or the callus on a supporting material and intermittently supplying and exhausting the liquid medium such that the tissue or the callus, or at least a part of the tuber-like tissue or the crown formed from the tissue or the callus, is intermittently immersed in the liquid medium.

5. A method according to any one of claims 2 to 4, wherein the liquid medium has a dissolved oxygen concentration (DO) of 9 ppm or more.

6. A method according to any one of the preceding claims which further comprises propagating the tuber-like tissue or crown by cutting a section therefrom, culturing the section to enlarge the tuber-like tissue or crown and, if required, repeating the propagation process.

7. A method according to claim 6, which comprises culturing the section under the conditions specified in any one of claims 2 to 5.

8. A method according to claim 6, which comprises culturing the section in a medium containing $10^{-8}$ M to $10^{-4}$ M cytokinin and $10^{-9}$ M to $10^{-5}$ M auxin.

9. A method according to any one of the preceding claims, which comprises the additional step of growing a plantlet by culturing the tuber-like tissue or crown, optionally in the presence of an antigibberellin.

10. A method according to claim 9, wherein the antigibberillin is ancymidol or uniconazol.

11. A method according to claim 9 or 10, which comprises growing the plantlet under the conditions specified in any one of claims 2 to 5.

12. A method according to claim 9, 10 or 11, which includes a rooting step.

13. A method according to claim 12, which comprises carrying out the rooting step in a medium containing $10^{-9}$ M to $10^{-5}$ M auxin.

14. A method according to any one of claims 9 to 13, which comprises growing the plantlet into a nursery plant by acclimatisation.

15. A method according to any one of the preceding claims, which comprises culturing the tissue or the callus to form a stock of the plant, dividing the stock into individuals each having at least one aerial part and root part, culturing the individuals to obtain stocks of the plant and, if required repeating the process.

16. A method according to claim 15, wherein the step of forming the stock of the plant by culturing the tissue or the callus comprises culturing a tissue or a callus of a plant belonging to the genus Asparagus to form a crown, culturing the crown to regenerate a plantlet and rooting the plantlet to form a stock of the plant.

# F I G. 1

# FIG.2

# FIG.3

## FIG.4

## FIG.5

# F I G. 6

OUT  OUT(FILTER)

FILTER

IN(AIR)

OUT

AIR PUMP

ASPARAGUS

SOLID MEDIUM

EP 0 375 218 A2

FIG. 7

AIR PUMP

FILTER

IN(AIR)

OUT

OUT(FILTER)

OUT

ASPARAGUS

POLYESTER WOOL

LIQUID MEDIUM

# FIG. 8

TUBE PUMP

TIMER

ELECTROMAGNETIC VALVE

CROWNS OF ASPARAGUS

STAINLESS WIRE GAUZE (SUPPORTING MATERIAL)

TIMER

# F I G. 9

MIST NOZZLE

AIR EXHAUST PORT

AIR INLET

ASPARAGUS

WATER
ABSORPTIVE
SUPPORT

POROUS SUPPORTING PLATE

EXHAUST PORT
OF
LIQUID MEDIUM

# F I G. 10

CUTTING

SHOOT

SECTION

FORMATION
OF
CROWN

CUTTING

PROPAGATION
OF
TUBER-LIKE
TISSUE

ROOTED INDIVIDUAL
(PLANT BODY)

# F I G. 11

# F I G. 12

SHOOT

CUTTING →

SECTION

FORMATION
OF
CROWN

SMALL STOCK

DIVISION

(CUTTING)

LARGE STOCK

EP 0 375 218 A2

# F I G.13

# F I G. 14

OUT     OUT(FILTER)

IN(AIR)

OUT

STAINLESS
WIRE GAUZE

LIQUID MEDIUM

TUBE PUMP

BUBBLING